# EUROPEAN PATENT APPLICATION

(11) **EP 2 269 465 A1**
(43) Date of publication of application: **05.01.2011**
(21) Application number: 10181020.8
(22) Date of filing: 03.05.2006
(51) Int. Cl.: A23C 9/123, A23C 9/13, A23L 1/30, A23L 1/308, A61K 35/74, C12N 1/20, C12R 1/01, C12R 1/225, C12R 1/23, C12R 1/25, C12R 1/46

(54) **Compositions comprising Bifidobacterium adolescentis**

(62) Divisional of application: 06756275.1
(71) Applicant: Probiotical S.p.a., 28100 Novara (NO) (IT)
(72) Inventor: Mogna, Giovanni, 28100 Novara (IT); Strozzi, Gian Paolo, 28100 Novara (IT)
(74) Representative: HOFFMANN EITLE

(57) **Abstract**

The present invention relates to a symbiotic composition that includes as a prebiotic, one or more polysaccharides, such as inulin and/or its analogues, and, as a probiotic, at least a bacterial strain, belonging to the genus *Bifidobacterium* capable of directly metabolizing said polysaccharide/s as such. Furthermore, the present invention relates to the use of said composition in a food and/or pharmaceutical field.

## Description

The aim of the present invention is a symbiotic composition including: as a prebiotic, one or more polysaccharides, such as inulin and/or its analogues, and, as a probiotic, at least a bacterial strain, belonging to the genus *Bifidobacterium* capable of directly metabolizing said polysaccharide/s as such. Furthermore, the aim of the present invention is the uses of said composition in a food and/or pharmaceutical field.

By the term "prebiotic", there are generally shown those substances or components of the diet (cell and reserve oligosaccharides and polysaccharides of the plants), neither digestible by the human digestive enzymes nor absorbable in the small intestine, which, once arrived in the colon, selectively stimulate the development and the activity of the microbial groups beneficial for the health of the individual. By the term "probiotic" there are shown, in turn, those living species-specific microorganisms which, when ingested or applied in a sufficient number, are able to induce in the consumer specific functional and beneficial effects on its state of health. The association of probiotics with prebiotics gives rise to compositions/products generally defined by the term "symbiotic".

In the formulation of an optimal symbiotic product, the presence of one or more probiotic bacterial strains belonging to the genes Bifidobacterium is considered fundamental. Bifidobacteria are anaerobic bacteria which form an important portion' of the intestinal bacterial microflora (consisting of an extremely complex mixed population, existing in the intestine in a quantity up to 10¹⁰-10¹² bacteria, belonging to at least 400 different species per gram of intestinal content). Said bacteria are able to contribute in a significant extent to the definition of an ecosystem favourable to a positive situation for the health of the guest. The nutritive and therapeutic probiotic effects of the bifidobacteria which, unlike the lactic bacteria, can- reach in the intestine concentration values up to 10⁹ CFU/g of intestinal content, can be bring back to the following main beneficial activities which obtain the unanimous consent of the international scientific community.
- Maintenance and/or restoration of a normal balance of the intestinal microflora thanks to the production of organic acids (lactic, acetic, propionic and formic) which reduce the pH of the intestine by inhibiting the growth of the undesired bacteria.
- Trophic action on the intestinal epithelial cells thanks to a direct action exerted on the enterocytes and the degradation and turnover of the parietal mucins.
- Barrier effect thanks to their ability in the adhesion to the enterocites by preventing in this way the adhesion of pathogenic species and phagocyte activation.
- Improvement of the lactose intolerance due to the capability of liberating beta-galactosidase in the small intestine.
- Synthesis of vitamins of the group B and amino acids (thiamine, riboflavin, vitamin B6 and K, alanine, valine, aspartic acid and threonine).
- Immunostimulating activity on the PBMCs (Pheripheal Blood Mononuclear Cells) with a production of antiinflammatory interleukin IL 10 and immuno-regulating IL 12, decrease of the pro-inflammatory interleukin IL 12, increase of the IgA antibody titer and transitory increase of the IFN-gamma.
- Increase of the absorption of some minerals and particularly of the calcium.
- Systemic antitumour activity through a direct removal of pro-carcinogen substances and at an intestinal topic level thanks to the trophic action exerted on the enterocytes.

As most of the intestinal bacteria capable of playing a positive role in the ambit of the intestinal ecosystem/ the bifidobacteria exert a saccharolytic action; that is, they obtain the energy required for their growth through the fermentation of the carbohydrates of endogenous and exogenous origin.

Generally, the bifidobacteria are able to use in the colon most of the non-digestible monosaccharides, disaccharides and oligosaccharides taken with the diet, or formed at the colon level following to the hydrolytic action on more complex glucidic molecules from enzymes secreted by other microbial groups forming the intestinal microflora.

On the contrary, the bifidobacteria do not result able to directly ferment the polysaccharides, polymeric carbohydrates composed of long chains of monosaccharides (more than 10 units).

The polysaccharides interesting as food are divided in two great classes: starchy and non-starchy.

The starch, the main energy reserve of the plants (particularly tubers and cereals) is a polysaccharide only composed of glucose units, existing in two different forms: amylose and amylopectin.

From the nutritional point of view, the cleavage of the starch, with a glucose formation, starts in the oral cavity thanks to the ptyalin action (salivary amylase) and is completed in the small intestine by the action of the pancreatic amylase.

Contrary to the starch, the non-starchy polysaccharides existing in fruit, vegetables, legumes and cereals, because of their specific chemical structure can not by hydrolysed by the human digestive enzymes and therefore, after the transit through the small intestine, they arrive integral in the colon. As a function of their solubility, the non-starchy polysaccharides are subdivides in water-soluble and non water-soluble.

Celluloses, hemicelluloses and lignins, which are not even degraded at the level of the large intestine because of the incapability from the intestinal microflora to produce specific enzymes, belong to the latter typology, mainly present in the cereals and some kinds of vegetables. This typology is generally defined as "non-starchy and non-cellulose polysaccharides".

On the contrary, pectins, hydrocolloids, rubbers and fructans belong to the water-soluble non-starchy polysaccharides.

The inulin, which is a polysaccharide consisting of a glucose molecule (in terminal position) and a varying number of fructose molecules (the average polymerization degree is 35), connected through glycosidic bonds, belongs to this latter typology.

The water-soluble polysaccharides and the non-digestible oligosaccharides, among which the fructooligosaccharides (FOS), constitute the main component of the food fiber (FA), which can be defined as that fraction of the foods of a plant origin that can not be digested by the digestive enzymes existing in our organism, is not absorbed in the small intestine and is partly or fully hydrolized in the colon through the intestinal microflora.

Although the concept of fiber is a recent acquisition, its nutritional and physiological importance in the regulation and regularization of the gastrointestinal functions is scientifically confirmed and universally accepted. In particular, the food fiber slows down the gastric transit (sense of repletion and therefore obesity prevention) and accelerates the intestinal one through- an increase of a fecal mass with a proper consistency suitable for the evacuant mechanism. Concerning these aspects, the action mechanism of the fiber is to be connected again to its capability of binding the water with a consequent increase of the fecal mass, in some cases more viscous for the gel properties of some fibers. By absorbing the water at the stomach level, there is an increase of the bolus allowing to reach the sense of repletion more quickly. The gel fraction slows down the emptying of the stomach and therefore also the digestion and the absorption in the intestine, where the feces will be more voluminous, less thick with a decrease of the pressure on the colon and an acceleration of the intestinal transit.

Thanks to the physical-chemical features and the prebiotic activity, the food fiber prevents and solves dysfunctions and pathological conditions which affect the intestine, such as constipation, diverticulosis and IBS (Irritable Bowel Syndrome).

Some food fibers, in addition to promote the absorption of the nutrients, are able to modulate some metabolic processes, proving to be effective in the prevention and therapy of the diabetes mellitus (hypoglycemic action) and the cardiovascular diseases (hypo-cholesterol action).

It is further known that the fiber, always thanks to its physical-chemical properties, plays a detoxifying and anticarcinogen action, binding the toxic substances and preventing in this way their absorption. In the ambit of the food fibers, some oligosaccharides (particularly the fructo-oligosaccharides, for simplicity FOS) and some non-starchy and non-cellulose polysaccharides (in particular inulin) proved of a particular interest as growth factors of specific microbial group beneficial for the health of the guest. FOSs have a polymerization degree, on average, from 2 to 10 (number of monomer units forming the polymer); therefore, they are not characterized by particularly long chains.

On the contrary, the inulin has a more heterogeneous polymerization degree, between 3 and 60, on average 35. Therefore, it includes both short-chain compounds and long-chain compounds.

Both FOSs and inulin are not hydrolysed in the stomach or absorbed in the small intestine, but they reach substantially intact the colon, where they are fermented through bacterial strains residing therein and promote the proliferation of bifidobacteria, confirming their effectiveness as prebiotics. Said fermentation is the result of a series of metabolic activities of the intestinal microorganisms. In fact, during their transit in the large intestine, the non-absorbed carbohydrates are mostly hydrolysed to their respective short-chain sugars (monomers and oligomers). Said short-chain sugars are in turn fermented to short-chain fatty acid (for simplicity, shortened SCFAs) and biomass from the existing bacterial flora, including bifidobacteria.

SCFAs are absorbed by the colon in an extent depending from the concentration and represent the most energetic source for the colonocytes, by supplying to the same up to 60-70% of their energetic requirement. Moreover, SCFAs stimulate the growth of the colon- rectal mucosa cells, delay the atrophy process of the mucosa and, specially the butyrate, decrease the risk of the onset of malignant transformations in the colon.

The metabolic process above-described takes place especially in the ascending part of the colon, where FOSs and short-chain sugars are rapidly consumed. On the contrary, the inulin has a much slower hydrolysis and consumption kinetic, therefore, its effect and the various metabolic activities, which result in an advantage for the health of the guest, are also ex-tending to the transverse and descending colon. However, only the smallest chains of the inulin can be fermented with an acceptable speed, during their transit in the colon; on the contrary, the long chains of the same (those with a polymerization degree, on the average, higher than 10-15) do hot result assailable from the intestinal bacterial flora (in particular from the bifidobacteria) and are largely eliminated with the feces.

It would be desirable to also obtain the fermentation of said long chains of the inulin in the transverse and descending colon, thus substantially increasing the production in the colon of bifidobacteria, with all the beneficial consequent effects. This possibility is not known so far.

There persists the need, therefore, of being able to completely use the inulin (not only its short chains) during the transit of the same along all the extension of the colon, so as to ensure an improvement of its bifidogenic effect, extending it to all said organ. The aim of the present invention is therefore to positively meet said need by obviating in this way to the drawbacks of the known art. This and other aims, which will result apparent from the following detailed description, have been attained by the Applicant, which has completely unexpectedly found that, contrary to what is known in the art, a very limited number of bacterial strains belonging to the genes Bifidobacterium are able to ferment the inulin as such, including its longest chains, thus allowing its use also in the transverse colon and the descending colon.

Therefore, a symbiotic composition including at least a polysaccharide non-digestible/absorbable by the organism and at least a bifidobacterium capable to ferment said polysaccharide is an aim of the present invention, as reported in the appended independent claim.

The use of said symbiotic composition for preparing food and/or pharmaceutical products for improving the bifidogenic activity in the organism is another aim of the present invention, as reported in the appended independent claim.

A food and/or pharmaceutical product containing the symbiotic composition of the present invention is a further aim of the present invention, as reported in the appended independent claim.

Preferred embodiments of the present invention are reported in the appended dependent claims. The symbiotic composition of the present invention includes:
- at least a prebiotic selected from non-starchy and non-cellulose long-chain polysaccharides, such as for example: fructanes (inulin), galactomannans (guar and rubbers), arabinogalactans (AG), arabinoxylanes (psyllium), galactans (agar and carrageenins), and/or their mixtures;
- at least a probiotic selected from bacterial strains belonging to the genes Bifidobacterium, capable to directly ferment said prebiotic as such, by using it as a carbon and energy source for its own growth and reproduction.

Preferably, said prebiotic is selected from inulin and arabinogalactan, and/or their mixtures. More preferably, said prebiotic is inulin. The spotting of the very little bacterial strains belonging to the genes Bifidobacterium, capable to directly ferment polysaccharide prebiotics as such, such as for example the inulin, has been completely unexpected.

In fact, during an in vitro experimentation, a high number of bacterial strains (about 50), representing most of the Bifidobacterium species of human and animal origin, has been growth in the same experimental conditions, in the presence of inulin as the only car-bon source.

Almost the entirety of the tested bacterial strains has confirmed, as it is known,- that is not able to ferment the inulin as such.

On the contrary, only four of said strains (not correlated therebetween, neither for the species, nor for the origin) have produced a high fermentation level of the prebiotic, by effectively reproducing themselves and giving rise to a significant SCFAs' production. The bacterial strains belonging- to the genes Bifidobacterium able to directly ferment the prebiotic are the following ones:
- *Bifidobacterium adolescehtis* ATCC 15703;
- *Bifidobacterium bifidum* DSM 20082;
- Bifidobacterium *thermophilum* ATCC 25866;
- Bifidobacterium *adolescentis* DSM 17103.

Accordingly, said at least one bacterial strain according to the present invention is selected from the four aforesaid strains and/or their mixtures. Preferably/ said strain is *Bifidobacterium adolescentis* DSM 17103 (deposited c/o the DSMZ, Deutsche Sammlung von Mikroorganismen und Zellkulturen GmbH, Braunsweig-Germany, on February 1, 2005, in the of Anidral srl).

*Bifidobacterium adolescentis* DSM 17103 is a bacillary Gram-positive, anaerobic, asporogenous and non-mobile microorganism which exclusively and typically uses the glucose through the fructose-6-phosphate shunt. It has been isolated from fecal material of a healthy individual and its optimal culture-growth conditions are: culture-substrate: TPY (trypticase g 10, phytone g 5, glucose g 10, tween 80 g 1, K₂HPO₄ g 2, MgCl₂·6H₂O. g 0.5, ZnSO₄·7H₂O g 0.25, CaCl₂·2H₂O g 0.15, L-cysteine hydrochloride monohydrate g 0.5, distilled water q.s. to 1,000 ml - pH after sterilization: 6.6 ± 0.1); temperature: 37 ± 1°C; incubation time with 1% inoculum: hours 16 ± 1.

In a preferred embodiment, the symbiotic composition of the present invention includes, as a prebiotic, inulin and, as a probiotic capable of directly fermenting the inulin as such, the bacterial strain *Bifidobacterium adolescentis* DSM 17103. The symbiotic composition of the present invention can further include FOS and/or other short-chain prebiotic substances.

Preferably, said other prebiotic substances are selected from: isomalt-oligosaccharides, modified and resistant starches, pectins, galacto-oligosaccharides, arabinogalactan, xylo-oligosaccharides, chitosan-oligo-saccharides, glucanmannan, beta-glucans, Konjac, guar, arabic, lemon, xanthan gums, polydextrose, D-tagatose. In a particularly preferred embodiment, the symbiotic- composition of the present invention includes inulin, the bacterial strain *Bifidobacterium adolescentis* DSM 17103 and FOS.

The symbiotic composition of the present invention can further include one or more bacterial probiotic strains unable to directly ferment the inulin as such, having complementary features, namely different intrinsic properties.

By way of absolutely non limiting example, said probiotic bacteria can be selected, among others, from those of the genes Lactobacillus, such as the species *Lactobacillus delbrueckii* subsp. *bulgaricus, Lactobacillus acidophilus, Lactobacillus gallinarum*, *Lactobacillus gasseri*, *Lactobacillus amylovorous*, *Lactobacillus johnsonii*, *Lactobacillus crispatus, Lactobacillus casei*, *Lactobacillus rhamnosus*, *Lactobacillus* paracasei, *Lactobacillus plantarum, Lactobacillus reuteri, Lactobacillus fermentum, Lactobacillus salivarius subsp. salivarius*, *Lactobacillus pentosus,* among those of the genes Bifidobacterium, such as *Bifidobacterium adolescentis*, *Bifidobacterium* animalis *subsp. animalis*, *Bifidobacterium animalis subsp. lactis*, *Bifido-bacterium bifidum*, *Bifidobacterium breve, Bifidobacterium infantis*, *Bifidobacterium longum*, *Bifidobacterium catenulatum*, *Bifidobacterium pseudocatenulatum* or from those of the genes Streptococcus, such as the species *Streptococcus salivarus subsp.* thermophilus. Preferably, said bacterial strain is selected from:
- *Lactobacillus acidophilus* LMG P-21381;
- *Lactobacillus paracasei* LMG P-21380;
- *Lactobacillus plantarum* LMG P-21021;
- *Lactobacillus pentosus* LMG P-21019;
- *Lactobacillus plantarum* LMG P-21020;
- *Lactobacillus plantarum* LMG P-21022;
- *Lactobacillus plantarum* LMG P-21023;
- *Bifidobacterium animalis subsp*. *lactis* LMG P-21384;
- *Streptococcus thermophilus* DSM 16506;
- *Streptococcus thermophilus* DSM 16507;
- *Bifidobacterium longum* DSM 16603;
- *Bifidobacterium breve* DSM 16604;
- *Lactobacillus rhamnosus* DSM 16605;
- *Lactobacillus delbrueckii* subsp. *bulgaricus* DSM 16606;
- *Lactobacillus delbrueckii subsp. bulgaricus* DSM 16607; and/or their mixtures.

In a particularly preferred embodiment, the symbiotic composition of the present invention includes inulin, the inulin-fermenting bacterial strain *Bifidobacterium adolescentis* DSM 17103 and the non inulin-fermenting bacterial strains *Bifidobacterium breve* DSM 16604 and *Lactobacillus plantarum* LMG P-21021.

In another particularly preferred embodiment, the symbiotic composition of the present invention includes inulin, the bacterial strain *Bifidobacterium adolescentis* DSM 17103, FOS and the non inulin-fermenting bacterial strains *Bifidobacterium breve* DSM 16604 and *Lactobacillus plantarum* LMG P-21021.

In another particularly preferred embodiment, the symbiotic composition of the present invention includes inulin, the bacterial strain *Bifidobacterium adolescentis* DSM 17103, FOS and the non inulin-fermenting bacterial strains *Bifidobacterium breve* DSM 16604 and *Lactobacillus paracasei* LMG P-21380.

The symbiotic composition of the present invention can further include also other active substances, such as antioxidants, hypoglycemics, hypocholesterolemics, immunostimulants and immunomodulatings, substances with an antiaterogenic, antimeteorism, antiulcer, laxative, antidiarrheal activity, vitamins, amino acids, mineral salts, enzymes, or proper excipients and/or additives, such as carriers, lubricants, dispersers, antiaggregating, flavourings, sweeteners, stabilizers, preservatives commonly used in the formulation pharmaceutical art.

By way of absolutely non limiting example, among the particularly preferred excipients and additives there may be mentioned maltodextrins, starch, tween, fragrances, such as those of mandarin, grapefruit, strawberry, bilberry, all fruits, calcium carbonate, magnesium stearate, talc, saccharose, glucose, acesulfame, saccharin, aspartame, ascorbic acid, parabens, glutamine, arginine, superoxide dismutase, glutathione. In the composition of the present invention, the prebiotic component is dosed in a quantity between 5% to 95% by weight, based on the total weight of the composition; preferably from 40% to 60%.

In turn, the probiotic component capable of fermenting the inulin is dosed in a quantity between 1% to 15% by weight, based on the total weight of the composition; preferably, from 5% to 10%.

The part lacking to 100% by weight of the composition, if any, consists of the additional substances above mentioned and/or proper additives and excipients. In a particularly preferred embodiment, the prebiotic component includes from 40% to 50% by weight of FOS and from 40% to 60% by weight of inulin based on the total quantity of said prebiotic component.

Representative, but not limiting examples of particularly preferred compositions according to the present invention.
A - example of a granular composition/sachet (g 5) to be dissolved in water before the administration
   - Inulin (prebiotic) mg 3,000
   - *B. adolescentis* DSM 17103 (probiotic) mg 250*
   - Maltodextrin (excipient) mg 1,420
   - Lemon fiber (stabilizing excipient) mg 330
   * equal to 25x10⁹ UFC (25 milliards)
B - example of a granular composition/sachet (g 5) to be dissolved in water before the administration
   - Inulin (prebiotic) mg 2,160
   - Fructo-oligosaccharides (FOS) (prebiotic) mg 1,440
   - *B. adolescentis* DSM 17103 (probiotic) mg 250*
   - Maltodextrin (excipient) mg 820
   - Lemon fiber (stabilizing excipient) mg 330
   * equal to 25x10⁹ UFC (25 milliards)
C - example of a granular composition/sachet (g 5) , to be dissolved in water before the administration
   - Inulin (prebiotic) mg 2,160
   - Fructo-oligosaccharides (FOS) (prebiotic) mg 1,440
   - *B*. *adolescentis* DSM 17103 (probiotic) mg 125⁽¹⁾
   - *B*. *breve* DSM 16604 (probiotic) mg 62.5⁽²⁾
   - *L*. *plantarum* LMG P-21021 (probiotic) mg 62.5⁽³⁾
   - Maltodextrin (excipient) mg 820
   - Lemon fiber (stabilizing excipient) mg 330
      (1) equal to 12.5x10⁹ UFC (12.5 milliards)
      (2) equal to 6.25x10⁹ UFC (6.25 milliards)
      (3) equal to 6.2.5x10⁹ UFC (6.25 milliards)
D - example of a granular composition/sachet (g 5), to be dissolved in water before the administration
   - Inulin (prebiotic) mg 3,000
   - *B. adolescentis* DSM 17103 (probiotic) mg 125⁽¹⁾
   - *B. breve* DSM 16604 (probiotic) mg 62.5⁽²⁾
   - *L. plantarum* LMG P-21021 (probiotic) mg 62.5⁽³⁾
   - Maltodextrin (excipient) mg 1,420
   - Lemon fiber (stabilizing excipient) mg 330
      (1) equal to 12.5x10⁹ UFC (12.5 milliards)
      (2) equal to 6.25x10⁹ UFC (6.25 milliards)
      (3) equal to 6.2.5x10⁹ UFC (6.25 milliards)
E - example of a granular composition/sachet (g 5), to be dissolved in water before the administration
   - Inulin (prebiotic) mg 2,160
   - Fructo-oligosaccharides (FOS) (prebiotic) mg 1,440
   - *B. adolescentis* DSM 17103 (probiotic) mg 125⁽¹⁾
   - *B. breve* DSM 16604 (probiotic) mg 62.5⁽²⁾
   - *L. paracasei* LMG P-21380 (probiotic) mg 62.5⁽³⁾
   - Maltodextrin (excipient) mg 820 - Lemon fiber (stabilizing excipient) mg 330
      (1) equal to 12.5x10⁹ UFC (12.5 milliards)
      (2) equal to 6.25x10⁹ UFC (6.25 milliards)
      (3) equal to 6.25x10⁹ UFC (6.25 milliards)

Particularly preferred compositions of the present invention are those for oral administration. Typical preferred formulation forms are, for example, capsules, compressed beads (in this case, to be administered together with the content of a prebiotic sachet, to be dissolved in water), solutions or suspensions ready to drink, powders or granules in sachets (to be suspended or dissolved in water or in nori-carbonated and soft drinks at the time of use) or analogous forms, effervescent formulations. The compositions of the present invention can also be formulated in a coated, lacquered, encapsulated or microencapsulated form, so as to result gastroresistant.

Said composition can also be formulated in a con- trolled-release form, so as to selectively release the active substances in the - gastrointestinal tract, in particular in the large intestine or the colon. Among the preferred embodiments of the present invention, there may be mentioned those symbiotic compositions arranged in a freeze-dried form. The freeze-drying of the composition is carried out by using techniques and equipments generally employed in the freeze-drying processes of pharmaceutical and/or food compositions. The compositions of the present invention are prepared in a traditional way by using, depending on the type of formulation that one wishes to prepare, preparative-techniques known to the skilled in the pharmaceutical art. By way of absolutely non limiting example, a granular formulation, to be suspended or dissolved in water at the time of use, will be prepared by intimately mixing the components of the composition (active substances, coadjuvants, excipients), reducing them to the desired granulometry and moisture degree, before packing them in single-dose sealed sachets.

In turn, a controlled-release composition will be prepared, for example, by microencapsulating or micro- coating the microgranulated mixture of the substances forming the formulation with opportune mixtures of biocompatible polymers (such as, for example, Eudragit of different type and structure) resistant to the gastric juices of the stomach and able to release said components after a proper residence time in the gastrointestinal tract, or at pH values typical of the colon. The microencapsulated mixture thus obtained will be used, for example, for the preparation of tablets, capsules or beads, depending on the selected commercial kind of presentation.

The dosage varies as a function of the different physical or pharmaceutical forms.

With reference to the prebiotic component, it is generally established such that the daily administration allows the ingestion of not less than 1 g of total food fiber, up to a maximum of 8 g pro die, preferably from 3 to 5 g.

By the term total food fiber, it is to be intended the sum of the quantities of inulin or other non-starchy and non-cellulose polysaccharide and the non-digestible oligosaccharides (FOS or analogues). Referring to the probiotic component, the dose pro die must be such that the ingestion of not less than 1x10⁹ UFC of active and viable cells of probiotic bacteria is allowed, up to a maximum of 300x10⁹ UFC; preferably, from 5 to 10x10⁹UFC.

Also in this case, in a similar way to the prebiotic component, the quantity of probiotic bacteria must be intended as a sum of all the species existing in the formulation. The following experimental section shows in detail some of the characteristic features of the present invention.

### Selection of the strains

47 bacterial strains, belonging to the genes Bifidobacterium, of a different origin and representing 11 different species, have been comparatively studied as for their growth capabilities by using as a single carbon source glucose, FOS, inulin, respectively. Many of said strains have been obtained from the ATCC and DSMZ collections; or they have been isolated (following methodologies known in the art) from the feces of healthy volunteers which, for a month, followed a diet without probiotics.

The strains have been anaerobically incubated in a MRS broth (DIFCO Laboratories) containing 0.5 gL⁻¹ of 1-cysteine·HCl. Colonies withdrawn after 24 hrs have been inoculated in 10 ml of a semisynthetic culture medium SM, containing 10 gL⁻¹ of glucose, FOS, inulin, respectively. The cultures have been anaerobically incubated at 37°C for 48 hrs and have been propagated for three times in the same medium.

The culture medium SM has the following composition (gL⁻¹): Casaminoacids (DIFCO Laboratories), 15; Yeast Nitrogen Base (DIFCO), 6.7; ascorbic acid, 10; sodium acetate, 10; (NH4J2SO4, 5; urea, 2; MgSO₄·7H₂O, 0,2; FeSO₄·7H₂O, 0.01; MnSO4·7H2O, 0.007; NaCl, 0.01; Tween 80, 1; cysteine, 0.5; pH adjusted at 7.0; autoclaved for 30 min at 110°C.

The growth of the strains has been determined by measuring the optical density at 600 nm (OD₆₀₀) and the pH at the end of the incubation.

All the strains have shown a significant growth both on glucose and on FOS (OD₆₀₀) values ≥ 1, on the average, between 1 and 2.5; final pH between 4 and 5). As for the inulin, only the four strains previously shown have demonstrated a significant growth on said carbon source (OD₆₀₀ values between 1 and 2; final pH of about 5).

On the contrary, all the other strains are not developed on said substrate (OD₆₀₀ values < 1, included, on the average, between 0 and 0.25; final pH on the average higher than 6). By way of example, for the strain *Bifidobacterium adolescentis* DSM 17103, an OD₆₀₀ value of about 1.9 and a final pH of about 5 have been detected.

Similar experiments have been carried out on fecal cultures (ideal for miming the in vivo environment in the colon) in the presence of FOS and inulin, respectively, as the only carbon sources. Also in this case, it has resulted that, while the bifidobacteria are generally able to use (and therefore develop themselves) the FOSs as a carbon source, only the four previously mentioned effectively use (and develop themselves) the long chains of the inulin. Further experiments, carried out by using known methodologies commonly used in the art, have involved the examination of the supernatant of the bacterial cultures for verifying if the bifidobacteria produce extra-cellular enzymes capable of hydrolysing FOSs and/or iniulin.

The examination has been carried out evaluating the hydrolytic activity towards the fructans from the supernatant of said bacterial cultures through HPAEC-PAD after 24 hrs of anaerobic incubation at 37°C. Said controls have shown that the capability of a Bifidobacterium strain of growing and developing itself using the inulin (in particular its long chains) as the only carbon source, can be correlated to the production from the same microorganism of extracellular enzymes (beta-fructofuranosidase) capable of hydrolysing the long chains of the fructans.

Also this fact further differentiates and characterizes the bifidobacteria of the present invention with respect to the other bifidobacteria. Accordingly, the presence of said extracellular beta-fructofuranosidase can be used as a useful base/marker for the targeting and the selection of new probiotic bacteria capable of fermenting the long chains of prebiotic saccharides. For illustrating the applicative potential of the compositions according to the present invention in a food field/ a medium formulation and the relative preparation of a probiotic yoghurt containing said composition are reported below.

To 1000 g of a traditional yoghurt, prepared according to any one of the known techniques, there are incorporated, after incubation:
- Inulin (mean polymerization degree of about 35): from 4 to 48 g
- *Bifidobacterium adolescentis* DSM 17103 (100x10⁹ UFC/g): from 0.5 to 10 g
- FOS: from 4 to 48 g
- *Bifidobacterium breve* DSM 16604 (100x10⁹ LTFC/g): from 0.5 to 10 g
- *Lactobacillus plantarum* LMG P-21021 (100x10⁹ UFC/g): from 0.5 to 10 g

After homogenization, the end product is packed in jars from 80 to 125 ml.

A jar with an average capacity (100 ml) preferably contains from 5x10⁹ UFC to 100x10⁹ UFC of all the probiotic strains/package and a total quantity of non-digestable, soluble food fibers between 10 to 80 g; preferably, from 30 to 50 g. The final pH is between 4.0 and 4.6. The product is stored at a temperature between +4 and +9°C. The shelf life is of 30 days.

The number of the probiotic strains at the end of the shelf life is ≥ 1x10⁹ UFC/jar; preferably, it is between 5x10⁹ UFC and 10x10⁹ UFC/jar.

Alternatively, it is also possible to accrete the above probiotic strains in the milk together with the symbiosis, instead of add them to the yoghurt after incubation. The end product thus obtained has the same features above described. The symbiotic composition previously described has been unexpectedly proposed as the desired solution to the technical problem of the present invention. The use of said symbiotic composition has in fact allowed to administer to the organism bacterial strains capable of metabolising the long chains of the inulin without the contribution of a preventive hydrolysis from the extracellular enzymes secreted by other microorganisms of the intestinal population. Said bacterial strains, administered in association with a high quantity of inulin, have allowed to sub-stantially increase the bifidogenic activity of the bifidobacteria extending it to all the colon, particularly to the transverse tract and the descending tract of the same.

The composition of the present invention can be administered in a variety of ways, depending oh the needs of the patient or the consumer.

In one of its preferred aspects, the present invention relates to food and/or pharmaceutical formulations containing said symbiotic composition, for carrying out the improvement and/or the restoration of the bifidogenic activity in the organism.

In a particularly preferred aspect, the symbiotic composition of the present invention is used for the preparation of food products based on milk, such as yoghurt, fermented milks, fresh cheeses and other food products, such as fruit-juices, functional drinks and integrators/ creams, desserts, puree, chocolate, stuffs and fillings used in the confectionery. By mere way of example, a particularly preferred embodiment foresees the preparation of a yoghurt having the following composition. A 125 ml package/jar of probiotic yoghurt contains:
- inulin g 3
- fructooligosaccharide (FOS) g 2
- *B. adolescentis* DSM 17103 ≥ 7x10⁹ UFC/jar
- *B*. *breve* DSM 16604 ≥ 7x10⁹ UFC/jar
- *L. plantarum* LMG P-21021 ≥ 7x10⁹ UFC/jar

The aforesaid populations of the probiotic strains relate to the end of the manufacturing, while at the end of the shelf life, the bacterial populations are ≥ 0.7x10⁹ UFC/jar for each of the strains.

A preferred embodiment refers to a symbiotic composition that includes at least a prebiotic which is selected from non-digestable long-chain polysaccharides and at least a probiotic bacterial strains which belongs to the genus *Bifidobacterium.* Said strain being capable of directly fermenting said prebiotic as such, using it as a carbon source, and being the *Bifidobacterium adolescentis* DSM 17103. In another preferred embodiment the prebiotic, which is comprised in said symbiotic composition, is selected from non-starchy and non-cellulose long-chain polysaccharides: fructans, inulin; galactomannans, guar/rubbers; arabinogalactans; arabinoxylanes, psyllium; galactans, agar, carrageenins; and/or their mixtures. Preferably, said prebiotic is selected from inulin, arabinogalactan and/or their mixtures.

In a preferred embodiment,said prebiotic is inulin.

In a preferred embodiment, said symbiotic composition further includes FOS and/or other short-chain prebiotics.

In a preferred embodiment, said symbiotic composition further includes at least another probiotic bacterial strain unable to directly fermenting the inulin as such, selected from: *Lactobacillus acidophilus* LMG P-21381, *Lactobacillus paracasei* LMG P-21380, *Lactobacillus plantarum* LMG P-21021, *Lactobacillus* pentosus LMG P-21019, *Lactobacillus plantarum* LMG P-21020, *Lactobacillus plantarum* LMG P-21022, *Lactobacillus plantarum* LMG P-21023, *Bifidobacterium animalis subsp. lactis* LMG P-21384, *Streptococcus thermophilus* DSM 16506, *Streptococcus thermophilus* DSM 16507, *Bifidobacterium longum* DSM 16603, *Bifidobacterium breve* DSM 16604, *Lactobacillus rhamnosus* DSM 16605, *Lactobacillus delbrueckii subsp. bulgaricus* DSM 16606, *Lactobacillus delbrueckii subsp. bulgaricus* DSM 16607 and/or their mixtures.

In a preferred embodiment said composition further includes the non inulin-fermenting bacterial strains *Bifidobacterium breve* DSM 16604 and *Lactobacillus plantarum* LMG P-21021.

In a preferred embodiment said composition further includes FOS.

In a preferred embodiment said composition further includes the non inulin-fermenting bacterial strains *Bifidobacterium breve* DSM 16604 and *Lactobacillus plantarum* LMG P-21021.

In a preferred embodiment said composition further includes the non inulin-fermenting bacterial strains *Bifidobacterium breve* DSM 16604 and *Lactobacillus paracasei* LMG P-21380.

In a preferred embodiment said composition further includes antioxidants, hypoglycemios, hypocholesterolemics, immunostimulants and immunomodulatings, substances with an antiaterogenic, antimeteorism, antiulcer, laxative, antidiarrheal activity, vitamins, amino acids, mineral salts, enzymes, excipients and/or additives, such as carriers, lubricants, dispersers, antiaggregating, flavourings, sweeteners, stabilizers, preservatives commonly used in the formulation pharmaceutical art.

In a preferred embodiment said composition is for the preparation of a food and/or pharmaceutical product for improving and/or restoring the bifidogenic activity in the organism.

A preferred embodiment relates to a food product for improving the bifidogenic activity in the organism, containing the aforementioned composition.

In a preferred embodiment said food product is a yoghurt.

A preferred embodiment relates to a pharmaceutical product for improving and/or restoring the bifidogenic activity in the organism that contains the aforementioned composition.

A preferred embodiment relates to *Bifidobacterium adolescentis* DSM 17103, deposited c/o the DSMZ (Deutsche Sammlung von Mikrorganismen und Zellkulturen GmbH, Braunsweig- Germany) on February 1, 2005.

A preferred embodiment relates to the use of *Bifidobacterium adolescentis* DSM 17103 for the preparation of a aforementioned composition.

A preferred embodiment relates to the use of *Bifidobacterium adolescentis* DSM 17103 for the preparation of a food and/or pharmaceutical product for improving and/or restoring the bifidogenic activity in the organism.

A preferred embodiment relates to a food and/or pharmaceutical product for improving and/or restoring the bifidogenic activity in the organism, said food includes *Bifidobacterium adolescentis* DSM 17103.

## Claims

1. A symbiotic composition comprising:
- at least a prebiotic selected from non-starchy and non-cellulose long-chain polysaccharides, and
- *Bifidobacterium adolescentis* DSM 17103.

2. The composition according to claim 1, wherein said prebiotic is selected from the group comprising fructans, inulin, galactomannans, guar/rubbers, arabinogalactans, arabinoxylanes, psyllium, galactans, agar, carrageenins and/or their mixtures.

3. The composition according to claim 2, wherein said prebiotic is selected from inulin, arabinogalactans and/or their mixtures.

4. The composition according to claim 3, wherein said prebiotic is inulin.

5. The composition according any one of the preceding claims, wherein it further includes fructooligosaccharides (FOS) and/or other short-chain prebiotics.

6. The composition according to claim 5, wherein it comprises *Bifidobacterium adolescentis* DSM 17103, inulin and fructooligosaccharides (FOS).

7. The composition according any one of the preceding claims, wherein it further includes at least another probiotic bacterial strain which is unable to directly fermenting the inulin as such, selected from: *Lactobacillus acidophilus* LMG P-21381, *Lactobacillus paracasei* LMG P-21380, *Lactobacillus plantarum* LMG P-21021, *Lactobacillus pentosus* LMG P-21019, *Lactobacillus plantarum* LMG P-21020, *Lactobacillus plantarum* LMG P-21022, *Lactobacillus plantarum* LMG P-21023, *Bifidobacterium animalis subsp. lactis* LMG P-21384, *Streptococcus thermophilus* DSM 16506, *Streptococcus thermophilus* DSM 16507, *Bifidobacterium longum* DSM 16603, *Bifidobacterium breve* DSM 16604, *Lactobacillus rhamnosus* DSM 16605, *Lactobacillus delbrueckii subsp. bulgaricus* DSM 16606, *Lactobacillus delbrueckii subsp. bulgaricus* DSM 16607 and/or their mixtures.

8. The composition according any one of the preceding claims, wherein it comprises inulin, the inulin-fermenting bacterial strain *Bifidobacterium adolescentis* DSM 17103 and the non inulin-fermenting bacterial strains *Bifidobacterium breve* DSM 16604 and *Lactobacillus plantarum* LMG P-21021.

9. The composition according any one of the preceding claims, wherein it comprises inulin, the bacterial strain *Bifidobacterium adolescentis* DSM 17103, fructooligosaccharides (FOS) and the non inulin-fermenting bacterial strains *Bifidobacterium breve* DSM 16604 and *Lactobacillus plantarum* LMG P-21021.

10. The composition according any one of the preceding claims, wherein it inulin, the bacterial strain *Bifidobacterium adolescentis* DSM 17103, fructooligosaccharides (FOS) and the non inulin-fermenting bacterial strains *Bifidobacterium breve* DSM 16604 and *Lactobacillus paracasei* LMG P-21380.

11. A composition according to any one of the claims 1-10 for use as a medicament for improving and/or restoring the bifidogenic activity in the organism.

12. A food product for improving the bifidogenic activity in the organism, containing a composition according to any one of the claims 1 to 10.

13. The food product according to claim 12, wherein said product is a yoghurt.

14. A bacterial strain *Bifidobacterium adolescentis* DSM 17103, deposited c/o the DSMZ (Deutsche Sammlung von Mikromicronorganismen und Zellkulturen GmbH, Braunsweig-Germany) on February 1, 2005.

15. Use of the bacterial strain according to claim 14, for the preparation of a food and/or pharmaceutical product for improving and/or restoring the bifidogenic activity in the organism.
